(19) Europäisches Patentamt
European Patent Office
Office européen des brevets

(11) Veröffentlichungsnummer: **0 411 419 A2**

(12) **EUROPÄISCHE PATENTANMELDUNG**

(21) Anmeldenummer: **90114014.5**

(22) Anmeldetag: **21.07.90**

(51) Int. Cl.5: **C07D 495/04**, A01N 43/90, A61K 31/38, //(C07D495/04, 333:00,311:00)

(30) Priorität: **03.08.89 DE 3925719**

(43) Veröffentlichungstag der Anmeldung: **06.02.91 Patentblatt 91/06**

(84) Benannte Vertragsstaaten: **BE CH DE ES FR GB IT LI NL**

(71) Anmelder: **BAYER AG**
**D-5090 Leverkusen 1 Bayerwerk(DE)**

(72) Erfinder: **Bertram, Heinz-Jürgen, Dr.**
**Nesselroder Strasse 27**
**D-5300 Bonn(DE)**
Erfinder: **Müller, Nikolaus, Dr.**
**Knipprather Strasse 98**
**D-4019 Monheim(DE)**
Erfinder: **Harder, Achim, Dr.Dr.**
**Auf dem Bruch 49**
**D-5090 Leverkusen 2(DE)**

(54) **Substituierte Thienopyranone, Verfahren zur ihrer Herstellung und ihre Verwendung zur Bekämpfung von Parasiten.**

(57) Die vorliegende Erfindung betrifft neue substituierte Thienopyranone der Formel I

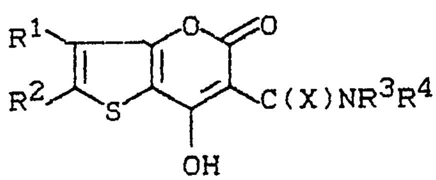

I

in welcher
X für O oder S steht,
$R^1$ und $R^2$ unabhängig voneinander für Wasserstoff, Halogen, CN, $NO_2$, Alkyl, Aralkyl, Aryl, Alkylcarbonyl, Alkoxycarbonyl stehen oder gemeinsam mit den angrenzenden C-Atomen einen carbocyclischen Ring bilden, der gegebenenfalls durch Heteroatome unterbrochen ist,
$R^3$ für gegebenenfalls substituiertes Alkyl oder Phenyl steht,
$R^4$ für Wasserstoff oder Alkyl steht,
$R^3$ und $R^4$ gemeinsam mit dem angrenzenden Stickstoffatom für einen heterocyclischen 5- oder 6-Ring stehen,
Verfahren zu ihrer Herstellung und ihre Verwendung zur Bekämpfung von Parasiten.

EP 0 411 419 A2

# SUBSTITUIERTE THIENOPYRANONE, VERFAHREN ZU IHRER HERSTELLUNG UND IHRE VERWENDUNG ZUR BEKÄMPFUNG VON PARASITEN

Die vorliegende Erfindung betrifft neue substituierte Thienopyranone, Verfahren zu ihrer Herstellung und ihre Verwendung gegen Parasiten.

3-Carbamoyl-4-hydroxycumarine und ihre Wirkung gegen Schädlinge sowie Parasiten sind bereits bekannt geworden (Deutsche Offenlegungsschrift DE-OS 3 012 642; 3 613 065; US-PB 587 786). Die Wirkung dieser Verbindungen ist jedoch nicht in jedem Fall befriedigend.

Die vorliegende Erfindung betrifft:

1. die neuen substituierten Thienopyranone der Formel I

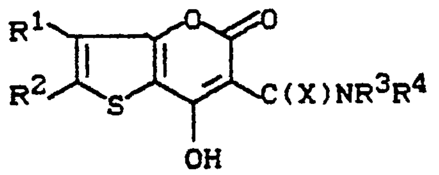

I

in welcher

X für O oder S steht,

$R^1$ und $R^2$ unabhängig voneinander für Wasserstoff, Halogen, CN, $NO_2$, Alkyl, Aralkyl, Aryl, Alkylcarbonyl, Alkoxycarbonyl stehen oder gemeinsam mit den angrenzenden C-Atomen einen carbocyclischen Ring bilden, der gegebenenfalls durch Heteroatome unterbrochen ist,

$R^3$ für gegebenenfalls substituiertes Alkyl oder Phenyl steht,

$R^4$ für Wasserstoff oder Alkyl steht,

$R^3$ und $R^4$ gemeinsam mit dem angrenzenden Stickstoffatom für einen heterocyclischen 5- oder 6-Ring stehen.

2. Verfahren zur Herstellung der neuen substituierten Thienopyranone der Formel I

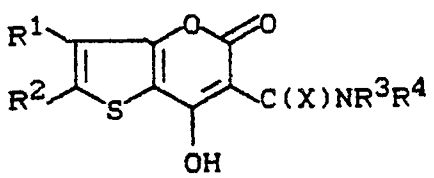

I

in welcher

X für O oder S steht,

$R^1$ und $R^2$ unabhängig voneinander für Wasserstoff, Halogen, CN, $NO_2$, Alkyl, Aralkyl, Aryl, Alkylcarbonyl, Alkoxycarbonyl stehen oder gemeinsam mit den angrenzenden C-Atomen einen carbocyclischen Ring bilden, der gegebenenfalls durch Heteroatome unterbrochen ist,

$R^3$ für gegebenenfalls substituiertes Alkyl oder Phenyl steht,

$R^4$ für Wasserstoff oder Alkyl steht,

$R^3$ und $R^4$ gemeinsam mit dem angrenzenden Stickstoffatom für einen heterocyclischen 5- oder 6-Ring stehen,

dadurch gekennzeichnet, daß man

a) für den Fall, daß in Formel I $R^4$ für Wasserstoff steht, Thienopyranone der Formel II

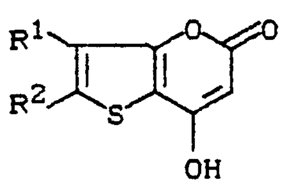

II

in welcher

$R^1$ und $R^2$ die oben angegebene Bedeutung haben

mit Iso(-thio-)cyanaten der Formel III

$X=C=N-R^3$ III

in welcher

$R^3$, X die oben angegebene Bedeutung haben,

umsetzt, oder

b) Verbindungen der Formel IV

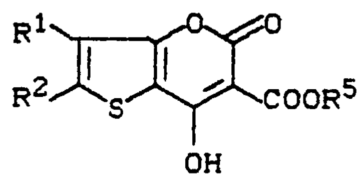

IV

in welcher

R1 und $R^2$ die oben angegebene Bedeutung haben und

$R^5$ für $C_{1-4}$-Alkyl steht,

mit Aminen der Formel V

H N $R^3$ $R^4$ V

in welcher

$R^3$ und $R^4$ die oben angegebenen Bedeutungen haben,

umsetzt, oder

c) Verbindungen der Formel II

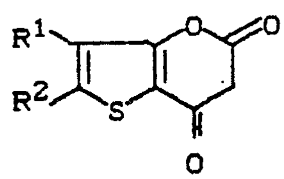

II

in welcher

R1 und $R^2$ die oben angegebene Bedeutung haben,

mit (Thio)-Carbamidsäurechloriden der Formel VI

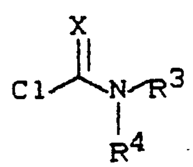

in welcher

X, $R^3$ und $R^4$ die oben angegebenen Bedeutungen haben,

umsetzt.

3. Verbindungen der Formel IV

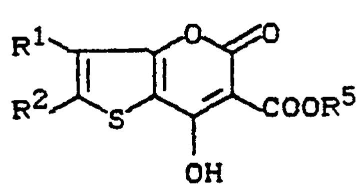

IV

in welcher

$R^1$, $R^2$, $R^5$ die unter (2) angegebenen Bedeutungen haben,

sind neu.

4. Verfahren zur Herstellung der Verbindungen der Formel IV gemäß (3) oben, dadurch gekennzeichnet,

daß man Verbindungen der Formel II

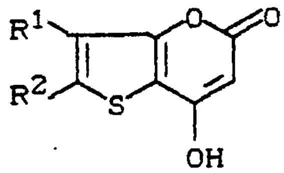

II

in welcher
$R^1$ und $R^2$ die oben angegebene Bedeutung haben,
mit Chlorameisensäureestern der Formel VII
$Cl\text{-}COOR^5$ VII
in welcher
$R^5$ für $C_{1-4}$-Alkyl steht,
umsetzt.

Bevorzugt sind Verbindungen der Formel I, in welcher
$R^1$ und $R^2$ für Wasserstoff, Halogen wie Fluor, Chlor, Brom, CN, $NO_2$, $C_{1-6}$-Alkyl, gegebenenfalls substituiertes Benzyl, $C_{1-4}$-Alkylcarbonyl, $C_{1-4}$-Alkoxycarbonyl, gegebenenfalls substituiertes Phenyl stehen, oder gemeinsam mit den angrenzenden C-Atomen für einen carbocyclischen 5-, 6- oder 7-gliedrigen Ring stehen, der gegebenenfalls auch durch O, N, S unterbrochen sein kann,
$R^3$ für gegebenenfalls substituiertes $C_{1-6}$-Alkyl, gebenenfalls substituiertes Phenyl steht,
$R^4$ für Wasserstoff, $C_{1-4}$-Alkyl steht,
$R^3$ und $R^4$ gemeinsam mit dem angrenzenden N-Atom für Pyrrolidin, Piperidin, Piperazin, Morpholin stehen.

Als Substituenten für Phenyl seien bevorzugt genannt: Alkyl mit vorzugsweise 1 bis 4, insbesondere 1 oder 2 Kohlenstoffatomen, wie Methyl, Ethyl, n.- und i.-Propyl und n.-, i.-, s.- und t.-Butyl; Alkoxy mit vorzugsweise 1 bis 4, insbesondere 1 oder 2 Kohlenstoffatomen, wie Methoxy, Ethoxy, n.-und i.-Propyloxy und n-, i.-, s.-und t.-Butyloxy; Alkylthio mit vorzugsweise 1 bis 4, insbesondere 1 oder 2 Kohlenstoffatomen, wie Methylthio, Ethylthio, n.-und i.-Propylthio und n.-, i.-, s.- und t.-Butylthio; Halogenalkyl mit vorzugsweise 1 bis 4, insbesondere 1 oder 2 Kohlenstoffatomen und vorzugsweise 1 bis 5, insbesondere 1 his 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; Alkylendioxy mit vorzugsweise 1 oder 2 Kohlenstoffatomen wie Methylendioxy oder Ethylendioxy; für halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy. Weitere Substituenten sind Halogen, vorzugsweise Fluor, Chlor, Brom und Jod, insbesondere Chlor und Brom; Cyano; Nitro; Dialkylamino mit vorzugsweise 1 bis 4, insbesondere 1 oder 2 Kohlenstoffatomen je Alkylgruppe, wie Dimethylamino, Diethylamino, Methyl-n.-butylamino; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Naphthylthio, die ihrerseits wieder durch einen der obengenannten Substituenten substituiert sein können.

Als Substituenten für Alkyl seien bevorzugt genannt: Halogen, insbesondere Chlor, Brom, Fluor, $C_{1-4}$-Alkoxy, insbesondere Methoxy, Ethoxy.

Besonders bevorzugt sind Verbindungen der Formel I, in welcher
$R^1$ und $R^2$ für Wasserstoff, Chlor, Brom, CN, $C_1$-$C_4$-Alkyl wie Methyl, Ethyl, i-Propyl, t-Butyl, gegebenenfalls substituiertes Phenyl, Acetyl, Methoxycarbonal, Ethoxycarbonyl stehen, oder gemeinsam mit den angrenzenden C-Atomen für einen 5- oder 6-Ring stehen,
$R^3$ für gegebenenfalls substituiertes Phenyl steht,
$R^4$ für Wasserstoff steht.

Besonders bevorzugte Substituenten für Phenyl sind: $C_1$-$C_4$-Alkyl, insbesondere Methyl, Ethyl, $C_1$-$C_4$-Alkoxy, insbesondere Methoxy, Ethoxy, $C_1$-$C_4$-Halogenalkoxy, insbesondere Trifluormethoxy, Fluorchlorethoxy, $C_1$-$C_4$-Halogenalkylthio, insbesondere Trifluormethylthio, Fluorchlormethylthio, $C_1$-$C_4$-Alkylthio, insbesondere Methylthio, Halogensulfonyl, insbesondere Fluorsulfonyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Methylendioxy oder Ethylendioxy, die gegebenenfalls durch Fluor oder Chlor substituiert sind, Halogen, insbesondere Fluor oder Chlor, CN, $NO_2$, Phenoxy, das gegebenenfalls durch einen der oben angegebenen Reste substituiert ist.

Ganz besonders bevorzugt sind Verbindungen der Formel I, in welcher

X für Sauerstoff steht,

$R^1$ für Wasserstoff steht,

$R^2$ für gegebenenfalls durch Halogen wie Chlor, Fluor, Brom, $C_{1-4}$-Alkyl wie Methyl, $C_{1-4}$-Halogenalkyl wie Trifluormethyl, $C_{1-4}$-Alkoxy wie Methoxy substituiertes Phenyl steht, oder $R^1$ und $R^2$ gemeinsam mit den angrenzenden C-Atomen für einen 5-Ring stehen,

$R^3$ für Phenyl steht, das gegebenenfalls durch Halogen wie Chlor, Fluor, Brom, $C_{1-4}$-Alkyl wie Methyl, $C_{1-4}$-Halogenalkyl wie Trifluormethyl, $C_{1-4}$-Alkoxy wie Methoxy, $C_{1-4}$-Alkylmercapto wie Methylmercapto, $C_{1-4}$-Halogenalkylmercapto wie Trifluormethylmercapto, $C_{1-4}$-Alkoxycarbonyl wie Methoxycarbonyl substituiert ist,

$R^4$ für Wasserstoff steht.

Im einzelnen seien folgende Verbindungen der Formel (I) genannt, in welcher die Reste $R^1$, $R^2$, $R^3$, $R^4$, X die angegebene Bedeutung besitzen:

| X | $R^1$ | $R^2$ | $R^3$ | $R^4$ |
|---|---|---|---|---|
| O | H | H | H | 4-Trifluormethyl-phenyl |
| O | H | H | H | 4-Trifluormethyl-mercaptophenyl |
| O | H | H | H | 3-Chlor-4-trifluor-methoxy-phenyl |
| O | H | H | H | 3-Fluorsulfonyl-phenyl |
| O | H | H | H | 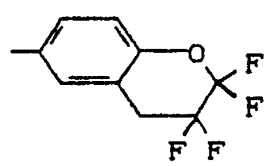 |
| O | H | H | H | 4-(3'-Trifluorme-thylphenoxy)-phenyl |
| O | H | H | H | 4-Fluor |
| O | H | H | H | 4-Methylmercapto-phenyl |

| X | $R^1$ | $R^2$ | $R^3$ | $R^4$ |
|---|---|---|---|---|
| O | $CH_3$ | H | H | 4-Chlorphenyl |
| O | $CH_3$ | H | H | 4-Methylphenyl |
| S | H | H | H | 4-Chlorphenyl |
| O | H | $CH_3$ | H | 4-Chlorphenyl |
| O | H | $CH_3$ | H | 4-Methylphenyl |
| O | F | H | H | 4-Chlorphenyl |
| O | F | H | H | 3,4-Dichlorphenyl |
| O | Cl | H | H | 4-Chlorphenyl |
| O | Cl | H | H | 3,4-Dichlorphenyl |
| O | H | F | H | 4-Chlorphenyl |
| O | H | F | H | 3,4-Dichlorphenyl |
| O | H | Cl | H | 3,4-Dichlorphenyl |
| O | H | Cl | H | 4-Chlorphenyl |
| O | H | $CH_3$ | H | 3,4-Dichlorphenyl |
| O | H | $CH_3$ | H | 2-Chlor-4-Trifluor-methylphenyl |
| O | H | $CH_3$ | H | 4-Trifluormercapto-phenyl |
| O | H | $CH_3$ | H | 4-Trifluormethoxy-phenyl |
| O | H | Br | H | 4-Chlorphenyl |
| O | H | Br | H | 4-Methylphenyl |
| O | Br | Br | H | 4-Chlorphenyl |
| O | Br | $CH_3$ | H | 4-Methylphenyl |
| O | Br | $CH_3$ | H | 4-Chlorphenyl |
| O | Br | $CH_3$ | H | 4-Trifluormethyl-mercaptophenyl |
| O | Br | $CH_3$ | H | 4-Trifluormethoxy-phenyl |
| O | Br | $CH_3$ | H | 2-Chlor-4-Trifluor-methylphenyl |

| X | $R^1$ | $R^2$ | $R^3$ | $R^4$ |
|---|---|---|---|---|
| O | H | Phenyl | H | 4-Methylphenyl |
| O | H | Phenyl | H | 4-Chlorphenyl |
| O | Phenyl | H | H | 4-Methylphenyl |
| O | Phenyl | H | H | 4-Chlorphenyl |
| O | $CH_3$ | Phenyl | H | 4-Methylphenyl |
| O | Phenyl | $CH_3$ | H | 4-Chlorphenyl |
| O | F | Cl | H | 4-Chlorphenyl |
| O | F | Cl | H | 3,4-Dichlorphenyl |
| O | F | F | H | 4-Chlorphenyl |
| O | F | F | H | 3,4-Dichlorphenyl |
| O | Cl | Cl | H | 4-Chlorphenyl |
| O | Cl | Cl | H | 3,4-Dichlorphenyl |
| O | $CH_3$ | $CH_3$ | H | 4-Chlorphenyl |
| O | $CH_3$ | $CH_3$ | H | 4-Methylphenyl |

Setzt man bei Verfahren 2a) als Thienopyranon der Formel II Thieno-[3,2]-7-hydroxy-pyran(5)on und als Verbindung der Formel III p-Tolylisocyanat ein, so läßt sich das Verfahren durch folgendes Formelschema darstellen:

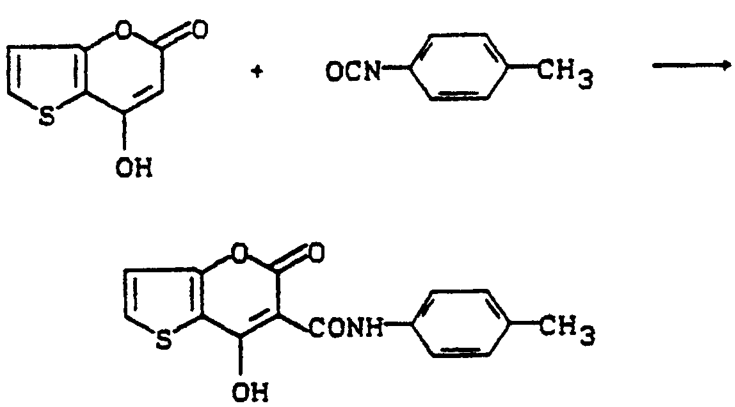

Die Verbindungen der Formel II sind teilweise neu.

Sie können nach an sich bekannten Verfahren aus 2-Aceto-3-hydroxythiophenen (VIII) hergestellt werden (T. Kralt, Recueil 86 (1967) S. 971-974).

Die Darstellung der 2-Aceto-3-hydroxythiophene gelingt durch Umsatz von 2,5-Dihydroxy-2,5-dimethyl-1,4-dithien mit Propidestern (vgl. T. Kralt, Recueil 86 (1967) S. 971-974).

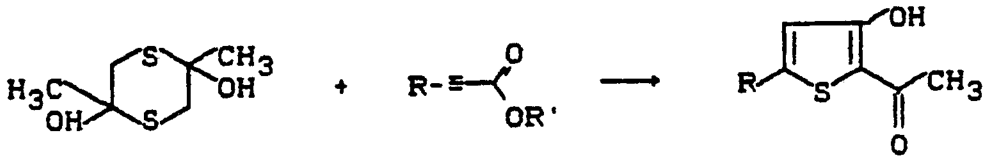

(VIII)

Alternativ dazu ist die Darstellung von 2-Aceto-3-hydroxythiophenen durch Umsatz von β-Halogensubstituierten α,β-ungesättigten Estern mit 2,5-Dihydroxy-2,5-dimethyl-1,4-dithion oder durch Umsatz von β-Thioxo-Estern mit α-Halogenaceton möglich.

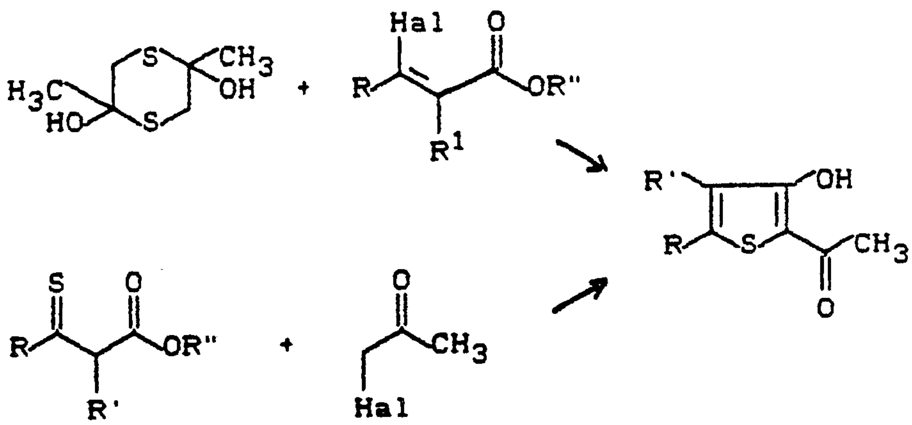

Die β-Thioxoster sind bekannt (F. Duus, Tetrahedron 28 (1972) S. 5923-5947.

Verbindungen der Formel III sind bekannt.

Bevorzugt werden Verbindungen der Formeln II und III eingesetzt, in denen X, $R^1$, $R^2$, $R^3$, $R^4$ die bei den Verbindungen der Formel I angegebenen bevorzugten und besonders bevorzugten Bedeutungen besitzen.

In einelzen seien folgende Verbindungen der Formel II genannt:

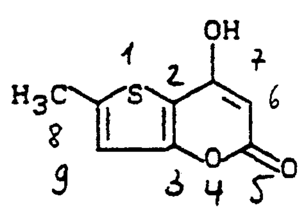

8-Methyl-thieno-[3,2]-7-hydroxypyran(5)on

8-Phenyl-thieno-[3,2]-7-hydroxypyran(5)on
8-Bromo-thieno-[3,2]-7-hydropyran(5)on
8-Chloro-thieno-[3,2]-7-hydroxypyran(5)on
8-Carboethoxy-thieno-[3,2]-7-hydroxypyran(5)on
8-Carbomethoxy-thieno-[3,2]-7-hydroxypyran(5)on
8-Ethyl-thieno-[3,2]-7-hydroxypyran(5)on
8-Methyl-9-bromo-thieno-[3,2]-7-hydroxypyran(5)on
8-Ethyl-9-bromo-thieno-[3,2]-7-hydroxypyran(5)on
8-Methyl-9-bromo-thieno-[3,2]-7-hydroxypyran(5)on
8-Ethyl-9-chloro-thieno-[3,2]-7-hydroxypyran(5)on
8-Methyl-9-chloro-thieno-[3,2]-7-hydroxypyran(5)on

9-Methyl-thieno-[3,2]-7-hydroxypyran(5)on
9-Ethyl-thieno-[3,2]-7-hydroxypyran(5)on
8-Bromo-9-methyl-[3,2]-7-hydroxypyran(5)on
8,9-Difluoro-thieno-[3,2]-7-hydroxypyran(5)on
8-Fluoro-thieno-[3,2]-7-hydroxypyran(5)on
9-Fluro-thieno-[3,2]-7-hydroxypyran(5)on
8,9-Dichlor-thieno-[3.2]-7-hydroxypyran(5)on
9-Bromo-thieno-[3,2]-7-hydroxypyran(5)on
8,9-Dibromo-thieno-[3,2]-7-hydroxypyran(5)on
8-Bromo-[3.2]-7-hydroxypyran(5)on
9-Chloro-[3,2]-7-hydroxypyran(5)on
8-(4′-Chlorphenyl)-thieno-[3,2]-7-hydroxypyran(5)on
8-(4′-Methoxyphenyl)-thieno-[3,2]-7-hydroxypyran(5)on
8-(3′-Chlorphenyl)-thieno-[3,2]-7-hydroxypyran(5)on
8-(2′-Chlorphenyl)-thieno-[3,2]-7-hydroxypyran(5)on
8-(2′,4′-Dichlorphenyl)-thieno-[3,2]-7-hydroxypyran(5)on
8-Phenyl-9-methyl-thieno-[3,2]-7-hydroxypyran(5)on
8-Phenyl-9-ethyl-thieno-[3,2]-7-hydroxypyran(5)on
8-Phenyl-9-bromo-thieno-[3,2]-7-hydroxypyran(5)on
8-Phenyl-9-chloro-thieno-[3,2]-7-hydroxypyran(5)on

In einzelnen seien folgende Verbindungen der Formel III genannt:
2,4-Dimethylphenylisocyanat
2,3-Dimethylphenylisocyanat
2,4,6-Trimethylphenylisocyanat
4-Chlor-2-methylphenylisocyanat
2-Chlor-4-methylphenylisocyanat
4-Bromphenylisocyanat
4-Methylphenyl-thioisocyanat

Verbindungen der Formeln II und III werden in Gegenwart von Verdünnungsmitteln und in Gegenwart von Basen sowie gegebenenfalls in Gegenwart weiterer Katalysatoren umgesetzt.

Als Basen seien genannt: Alkali-, Erdalkalialkoholate und tertiäre Amine. Besonders bevorzugt seien folgende Basen genannt: Triethylamin, Pyridin, Picoline, Trimethylamin, N-Methylmorpholin, N-Ethylpyrrolidin, Diazabicyclo(4,3,0)-undecen (DBU), 1,4-Diaza-bicyclo-2,2,2-octan (DABCO), Diazabicyclo(3,2,0)nonen (DBN).

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Katalysatoren kommen die bei Umsetzungen mit Isocyanaten üblichen Katalysatoren infrage. Als solche seien genannt: Metallkatalysatoren des Zn, Sn, Pb wie Dibutylzinndilaurat, Dibutylzinndioxid, Zinnoctoat, Bleioctoat, Zinkoctoat, Zinkchlorid, Zinkacetat.

Die Reaktion wird zwischen 0 und 150 °C bevorzugt zwischen 20-50 °C durchgeführt. Man arbeitet vorzugsweise unter Normaldruck.

Die Verbindungen der Formeln II und III werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit verdünnter Säure, Abfiltrieren des Produkts oder Abtrennen der organischen Phase und Abdestillieren des Lösungsmittels.

Setzt man bei Verfahren 2b) als Verbindung der Formel IV Dichlorthieno-[3,2]-7-hydroxy-6-ethoxycarbonyl-pyran(5)on und als Verbindung der Formel V 4-Trifluormethoxyanilin ein, läßt sich das Verfahren durch folgendes Formelschema darstellen:

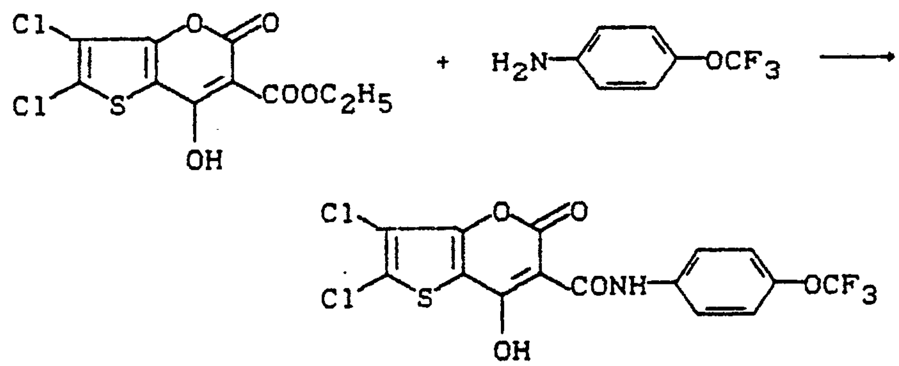

Die Verbindungen der Formel IV sind neu. Ihre Herstellung wird weiter unten beschrieben Verbindungen der Formel V sind bekannt. Bevorzugt werden Verbindungen der Formeln IV und V eingesetzt, in denen $R^1$, $R^2$, $R^3$, $R^4$ die bei den Verbindungen der Formel I angegebenen bevorzugten und besonders bevorzugten Bedeutungen besitzen.

Im einzelnen seien folgende Verbindungen der Formel IV genannt:
6-Carboethoxy-thieno-[3,2]-pyran-5-on
6-Carbomethoxy-thieno-[3,2]-pyran-5-on

Im einzelnen seien folgende Verbindungen der Formel V genannt:

4-Trifluormethoxy anilin, 4-Trifluormethylmercaptoanilin, 3-Chlor-4-trifluormethoxy anilin, 3-Chlor-4-trifluormethylmercaptoanilin, 3-Nitro-4-trifluormethoxyanilin, 4-(1,1,2,2-Tetrafluorethoxy)-anilin, 2,6-Dichlor-4-trifluormethylmercapto-anilin, 4-Amino-4′-trifluormethyl-diphenylether, 4-Amino-3′-trifluormethyldiphenylether;

Die Umsetzung der Verbindungen der Formel IV und V erfolgt vorzugsweise in Gegenwart von Verdünnungsmitteln sowie in Gegenwart von Basen.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid, ferner Alkohole wie Methanol, Ethanol, Propanol, Butanol.

Als Basen seien genannt Alkali- und Erdalkalihydroxide, Alkali- und Erdalkalialkoholate, insbesondere Natriummethylat oder -ethylat.

Die Reaktion wird zwischen 50 und 150°C bevorzugt zwischen 60-110°C durchgeführt. Man arbeitet vorzugsweise unter Normaldruck.

Die Verbindungen der Formeln VI und VII werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase und Abdestillieren des Lösungsmittels.

Setzt man bei Verfahren 4 als Verbindung der Formel II 2-Methyl-7-hydroxy-thieno[3,2]propan-5-on und als Verbindung der Formel VI Chlorameisensäuremethylester ein, so läßt sich Verfahren 4 durch folgendes Formelschema darstellen:

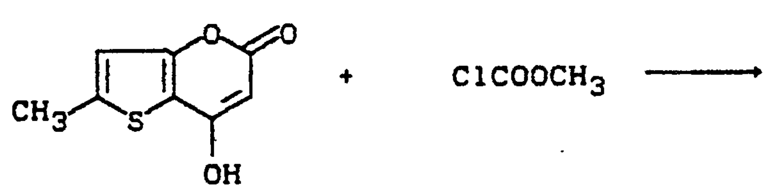

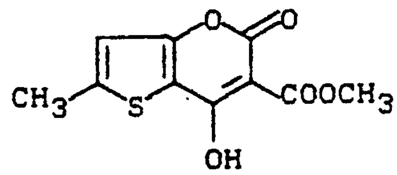

Verbindungen der Formel II sind teilweise neu (s.o.). Verbindungen der Formel VI sind bekannt.

Bevorzugt werden Verbindungen der Formel II eingesetzt, in denen $R^1$ und $R^2$ die bei den Verbindungen der Formel I angegebenen bevorzugten und besonders bevorzugten Bedeutungen besitzen.

Im einzelnen seien die obengenannten Verbindungen der Formel II erwähnt. Im einzelnen seien folgende Verbindungen der Formel VI erwähnt:
Chlorameisensäuremethylester
Chlorameisensäureethylester

Die Umsetzung erfolgt bei Temperaturen von 20 - 200 °C, bevorzugt bei 50 - 150 °C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Alkohole wie Methanol, Ethanol, Isopropanol, Butanol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecen(DBU), 1,4-Diazabicyclo(2,2,2)octan (DABCO), Diazabicyclo(3,2,0)nonen (DBN), Ethyl-diisopropylamin.

Die Verbindungen der Formeln II und VI werden im Verhältnis 1:1 bis 1:1,5 zueinander eingesetzt. Bevorzugt ist ein etwa äquimolares Verhältnis. Die Basen werden in etwa äuqimolar, bezogen auf die Verbindungen der Formel VI, eingesetzt.

Nach erfolgter Umsetzung wird das Verdünnungsmittel zum Teil (zu ca. 50 %) abdestilliert, der Rest mit wässriger Säure versetzt und die Verbindungen der Formel VI in an sich bekannter Weise isoliert, indem sie mit einem geeigneten Lösungsmittel, z.B. Ether oder Methylenchlorid, extrahiert werden.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp.,

Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln;
Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt: Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Beispiel A

In vitro Nematodentest

Caenorhabditis elegans

$10^{-4}$ g Wirkstoff werden in 1 ml Wasser oder 0,1 ml Dimethylsulfoxid (DMSO) gelöst. Diese Lösung wurde auf eine Replica-Platte gegeben. Dazu wurden 2 ml einer E.coli-Suspension gegeben, zu der man 10-20 weibliche Tiere oder Larven von Caenorhabditis elegans in 0,5 ml sterile M9 Pufferlösung gegeben

hat. Die E.coli-Suspension wurde hergestellt indem man 300 ml einer Übernachtkultur eines Uracil-bedürftigen E.coli-Stammes mit 1,8 l steriler M9 Pufferlösung versetzte.

Der Versuchsansatz wurde 7 Tage bei 22 °C inkubiert und danach ausgewertet. Es wurde bewertet inwieweit der Wirkstoff die Vermehrung beeinträchtigt und die Konzentration angegeben bei der die Vermehrung verhindert wird. Dabei wurden folgende Ergebnisse erhalten:

Tabelle A

| In-vitro Nematodentest | |
|---|---|
| Caenorhabditis elegans | |
| Wirkstoff Beispiel Nr. | effektive Dosis (µg/ml) |
| 1 | 100 |
| 2 | 100 |
| 3 | 100 |
| 5 | 100 |
| 6 | 100 |
| 7 | 100 |
| 8 | 100 |
| 9 | 100 |
| 10 | 100 |
| 11 | 100 |
| 12 | 100 |
| 13 | 100 |
| 14 | 100 |
| 15 | 100 |

Beispiel B

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 2 | 10 |
| 13 | 10 |

Beispiel C

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Würmer vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 2 | 10 |
| 10 | 10 |
| 13 | 25 |

Herstellungsbeispiele

Beispiel 1

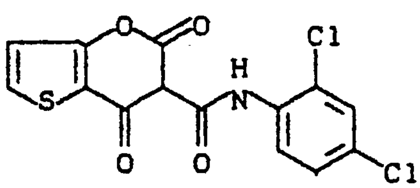

2,55 g (0,015 Mol) Thieno-[3,2]-7-hydroxypyran(5)on werden in 20 ml DMSO aufgenommen. Dann werden langsam 1,5 g Triethylamin und danach 2,82 g (0,015 Mol) 2,4-Dichlorphenylisocyanat zugegeben.

Man rührt 12 Stunden bei Raumtemperatur und gießt dann in 75 ml Wasser, das zuvor mit 4,5 ml konz. Salzsäure angesäuert wurde, ein.

Man rührt 30 Minuten bei 0 °C, saugt ab und verrührt den Niederschlag mit 100 ml Methanol-Methylenchloridlösung (1:1). Das Produkt wird abgesaugt und getrocknet.

Ausbeute: 4,3 g (80,5 %)

Fp.: 261 °C

IR (KBr): 1705; 1590; 1540; 1045

NHR (DMSO): 7,38 d (1); 7,5 dd (1); 7,63 d (1);

8,1 d (1); 8,3 dd (1); 11,65 s (1)

Analog werden erhalten:

Analog werden erhalten:

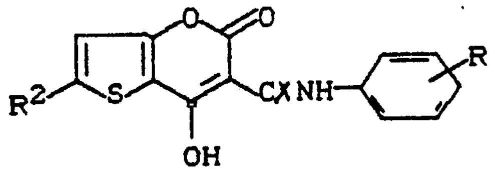

| Bsp. Nr. | $R^2$ | R | X | Fp.°C |
|---|---|---|---|---|
| 2 | H | 4-Cl | O | 220 |
| 3 | H | 3-Cl, 4-$CF_3$ | O | 230 |
| 4 | H | 2,4,5-$Cl_3$ | O | 254-255 |
| 5 | H | 2,3,4,5,6-$Cl_5$ | O | 251 |
| 6 | H | 4-$OC_2H_5$ | O | 183-184 |
| 7 | H | 3-Cl | O | 210-211 |
| 8 | H | 3,4-$Cl_2$ | O | 249-250 |
| 9 | H | 3-$CF_3$ | O | 190 |
| 10 | H | 4-$SCF_3$ | O | 218-220 |
| 11 | H | 3-Cl, 4-$SCF_3$ | O | 204-205 |
| 12 | H | 3-Cl, 4-$CH_3$ | O | 203-204 |
| 13 | H | 4-$CH_3$ | O | 186 |
| 14 | 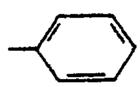 | 4-Cl | O | 257-258 |
| 15 | H | 4-$OCF_3$ | O | 203-204 |
| 16 | 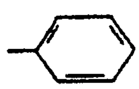 | 4-$CH_3$ | O | 274 |
| 17 | $CH_3$ | 4-Cl | O | 214 |
| 18 | H | 3 Cl, 4-$OCF_3$ | O | 167 |

| Bsp.Nr. | $R^2$ | X | R | Fp. °C |
|---|---|---|---|---|
| 19 | $CH_3$ | O | $3-CF_3$ | 202-203 |
| 20 | H | O | $3-SO_2F$ | 230 |
| 21 | $CH_3$ | O | $4-OCF_3$ | 210 |
| 22 | $CH_3$ | O | $3-Cl,4-OCF_3$ | 214 |
| 23 | $CH_3$ | O | $4-SCF_3$ | 221 |
| 24 | $CH_3$ | O | $3-SO_2F$ | 254-255 |
| 25 | $CH_3$ | O | $3-Cl,4-SCF_3$ | 226 |
| 26 | $CH_3$ | O | $3-Cl,4-SF_2Cl$ | 216 |
| 27 | $CH_3$ | O | 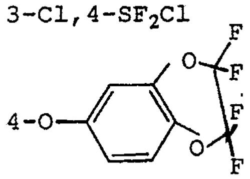 | 212 |
| 28 | $CH_3$ | O | 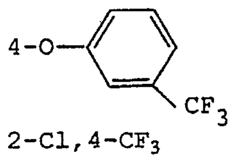 | 208 |
| 29 | $CH_3$ | O | $2-Cl,4-CF_3$ | 240 |
| 30 | $CH_3$ | O | $2,4,5-Cl_3$ | 231 |
| 31 | $CH_3$ | O | 3-Cl | 211 |
| 32 | $CH_3$ | O | $4-CH_3$ | 238 |
| 33 | $CH_3$ | S | H | 181-182 |
| 34 | $CH_3$ | O | 4-Cl | 221-222 |
| 35 | $CH_3$ | O | $2,4-Cl_2$ | 225-226 |
| 36 | $CH_3$ | O | $3,4-Cl_2$ | 227 |
| 37 | $CH_3$ | O | $2,4,5,6-Cl_5$ | 180 |
| 38 | $CH_3$ | O | $3-Cl,4-SCF_3$ | 224 |
| 39 | $CH_3$ | O | $3-NO_2$ | 240 |
| 40 | $CH_3$ | O | $4-OC_2H_5$ | 216 |
| 41 | H | S | 4-Cl | 207-208 |
| 42 | H | O | $3-NO_2$ | 227-228 |

| Bsp.Nr. | $R^2$ | X | R | Fp. °C |
|---|---|---|---|---|
| 43 | 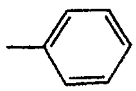 | O | 2-Cl,4-$CF_3$ | 198-199 |
| 44 | H | O | 3-Cl,4-$SCF_3$ | 178-180 |
| 45 | H | O | 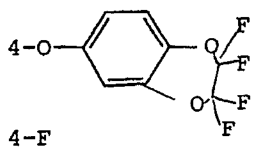 | 205 |
| 46 | H | O | 4-F | 220-221 |
| 47 | H | O | 4-Br | 228-229 |
| 48 | $CH_3$ | O | 4-F | 190-191 |

Beispiele für die Herstellung der Ausgangsprodukte:

a) Herstellung von Thieno-[3,2]-7-hydroxypyran-5-on

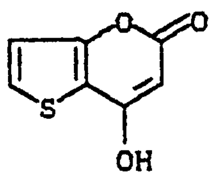

2,07 g (0,00966 Mol) 2-Ethoxycarbonylaceto-3-hydroxythiophen werden mit 50 ml absolutem Xylol für 1 Stunde am Rückfluß gekocht. Währenddessen wird langsam das Xylol abgezogen, das Produkt fällt dabei als Feststoff aus und wird abgesaugt.

Ausbeute: 0,4 g (22 %)

Fp.: 223-225° C

Analog werden dargestellt:

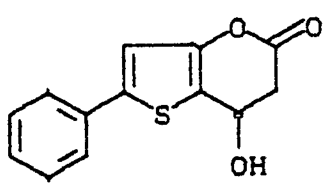

$C_{13}H_8O_3S$ Fp.: 214-215° C

NMR ($CDCl_3$): 5,6 s (1), 7,2 s (1), 7,4-7,6 m (s)

IR (KBr): 1640; 1560; 1480; 1400

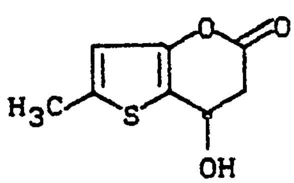

$C_8H_6O_3S$ Fp.: 210-212 °C
NMR ($CDCl_3$): 1,6 s (3), 7,3 s (1)
IR (KBr): 1680; 1560; 1510; 1330
MS: 182 (65 %); 140 (100 %); 112 (30 %)

b) Herstellung von 3-Ethoxycarbonylaceto-3-hydroxythiophen

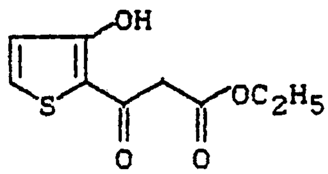

4,8 g (0,0337 Mol) 2-Aceto-3-hydroxythiophen werden in 150 ml Diethylcarbonat auf 90-100 °C erwärmt. Dann werden in kleinen Stücken 5 g Natrium zugegeben. Es wird 15 Stunden bei 90-100 °C nachgerührt. Zur Zersetzung von Natriumresten wird etwas Ethanol zugegeben. Zur Aufarbeitung wird 2 mal mit 75 ml Wasser ausgeschüttelt, dann die wäßrige Phase mit 10 ml konz. HCl angesäuert, 3 mal mit je 50 ml Ether ausgeschüttelt, getrocknet und im Vakuum eingeengt. Zur Rohaufreinigung wird im Vakuum destilliert und die Fraktion zwischen 70 und 100 °C (0,5 Torr, Edukt) sowie zwischen 113 und 118 °C (0,3 Torr, Produkt) aufgefangen.

Zur Reinigung wird mit Essigester/Cyclohexan 1:1 gesäult.
Ausbeute: 0,5 g (6,9 %).

c) Herstellung von 2-Aceto-3-hydroxythiophen

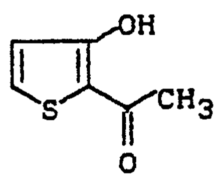

4,65 g $NaOCH_3$ werden in 100 ml Toluol suspendiert. Dann wird eine Suspension von 7,6 g (0,0843 Mol) Acetonylmercaptan in 150 ml Toluol zugegeben. Danach werden 7,1 g (0,0843 Mol) Methylpropiolat zugetropft. Man kocht 1 Stunde am Rückfluß. Nach dem Abkühlen wird eine Lösung aus 4,5 ml konz. $H_2SO_4$ in 120 ml Wasser zugetropft. Die Toluolphase wird abgetrennt und 3 mal mit je 50 ml Wasser gewaschen, getrocknet und eingeengt. Der Ansatz wird destilliert, die Rohfraktion zwischen 85 °C und 90 °C bei 12 Torr wird aufgefangen. Gewicht des Rohproduktes: 6 g. Das Rohprodukt wird in 48 ml 1 n NaOH gegossen und mit Ether ausgeschüttelt. Die wäßrige Phase wird mit 2 n HCl angesäuert und mit Ether extrahiert. Nach dem Trocknen und Einengen wird mit Ether verrieben.
Ausbeute: 1,2 g (10 %)
Fp.: 48-50 °C

Analog wurde dargestellt:

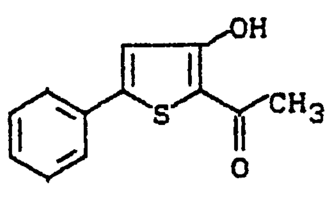

$C_{12}H_{10}O_2S$ Fp.: 93-94 °C
NMR ($CDCl_3$): 2,5 s (3); 2,7 s (3); 6,65 s (1)

IR (KBr): 1600; 1480; 1420; 1370; 1340

Herstellung von 5-Methyl-hydroxythiopen

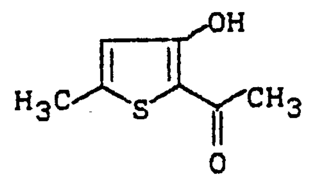

$C_7H_8O_2S$ 156,3 g/mol

Verfahren a)

42,1 (0,78 Mol) Natriummethylat werden in 1000 ml Toluol suspendiert. Dann werden 70,3 g 2,5-Dihydroxy-2,5-dimethyl-1,4-dithian (0,78 Mol) und 190 g (1,28 Mol) β-Chlor-crotonsäureethylester zugegeben.

Anschließend wird für 2 Stunden rückflüssiert. Man kühlt ab und gibt eine Lösung von 41,7 ml konz. $H_2SO_4$ in 1100 ml Wasser zu der Reaktionsmischung.

Die organische Phase wird abgetrennt, die wäßrige Phase mit Toluol nachextrahiert. Die vereinigten organischen Phasen werden mit Wasser gewaschen und anschließend über $Na_2SO_4$ getrocknet.

Nach dem Abfiltrieren des Trockenmittels wird das Lösungsmittel abgezogen. Der Rückstand wird in 450 ml 1 N NaOH-Lösung eingenommen und einmal mit Ether extrahiert. Die wäßrige Phase wird mit 2 N HCl angesäuert und mit Ether extrahiert. Die organischen Phasen werden über $Na_2SO_4$ getrocknet. Nach dem Abtrennen des Trockenmittels und Abziehen des Lösungsmittels wird der Rückstand durch Verreiben mit Ether auskristallisiert.

Ausbeute: 20 g (16 %)

Fp: 74 - 75 °C

IR (KBr): 1490; 1440; 1220; 1040

NMR (CD $Cl_3$): 2,3 s (3); 2,45 d (3); 6,5 d (1); 11,7 s breit (1)

MS: 156 (100 %); 141 (100 %)

Verfahren b)

Zu einer Lösung von 0,025 Mol Natriumhydrid in 50 ml absolutem Benzol wird unter Stickstoffatmosphäre eine Lösung von 2,92 g (0,02 Mol) β-Thioacetessigsäureethylester, gelöst in 10 ml absolutem Benzol getropft.

Anschließend wird für 30 Minuten rückflussiert.

Dann werden 2,78 g (0,03 Mol) Chloraceton, gelöst in 20 ml absolutem Benzol zugetropft und es wird für weitere 3 Stunden rückflüssiert. Danach wird der Ansatz auf Eiswasser gegossen und angesäuert. Die organische Phase wird abgetrennt und das Lösungsmittel abgezogen.

Der Rückstand wird in 100 ml absolutem DMSO aufgenommen. Unter Stickstoffatmosphäre werden 0,03 Mol Natriumhydrid zugegeben, anschließend wird der Ansatz für 2 Stunden gerührt. Danach wird der Ansatz in Wasser eingetragen und angesäuert. Die wäßrige Phase wird mit Ether extrahiert und die organische Phase über $Na_2SO_4$ getrocknet.

Nach dem Abziehen des Lösungsmittels wird das Produkt durch Chromatographie gereinigt (Laufmittel: Essigester/Cyclohexan 1 : 1).

Ausbeute: 470 mg (15 %)

Analog wurde dargestellt:

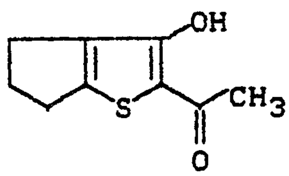

$C_9H_{10}O_2S$ (blaßgelbes Öl)
IR (KBr):
NMR ($CDCl_3$): 2,3 s (3); 2,4 - 2,5 m (2); 2,6 - 2,7 m (2); 2,85 - 2,95 m (2); 11,8 s (1)
MS: 182 (56 %); 167 (100 %)

**Ansprüche**

1. Substituierte Thienopyranone der Formel I

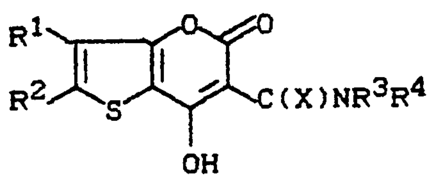

I

in welcher
X für O oder S steht,
$R^1$ und $R^2$ unabhängig voneinander für Wasserstoff, Halogen, CN, $NO_2$, Alkyl, Aralkyl, Aryl, Alkylcarbonyl, Alkoxycarbonyl stehen oder gemeinsam mit den angrenzenden C-Atomen einen carbocyclischen Ring bilden, der gegebenenfalls durch Heteroatome unterbrochen ist,
$R^3$ für gegebenenfalls substituiertes Alkyl oder Phenyl steht,
$R^4$ für Wasserstoff oder Alkyl steht,
$R^3$ und $R^4$ gemeinsam mit dem angrenzenden Stickstoffatom für einen heterocyclischen 5- oder 6-Ring stehen.

2. Verfahren zur Herstellung der neuen substituierten Thienopyranone der Formel I gemäß Anspruch 1

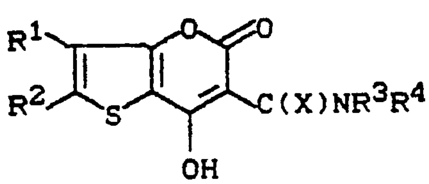

I

in welcher
X für O oder S steht,
$R^1$ und $R^2$ unabhängig voneinander für Wasserstoff, Halogen, CN, $NO_2$, Alkyl, Aralkyl, Aryl, Alkylcarbonyl, Alkoxycarbonyl stehen oder gemeinsam mit den angrenzenden C-Atomen einen carbocyclischen Ring bilden, der gegebenenfalls durch Heteroatome unterbrochen ist,
$R^3$ für gegebenenfalls substituiertes Alkyl oder Phenyl steht,
$R^4$ für Wasserstoff oder Alkyl steht,
$R^3$ und $R^4$ gemeinsam mit dem angrenzenden Stickstoffatom für einen heterocyclischen 5- oder 6-Ring stehen,
dadurch gekennzeichnet, daß man
a) für den Fall, daß in Formel I $R^4$ für Wasserstoff steht, Thienopyranone der Formel II

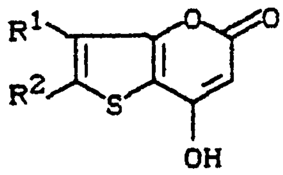

II

in welcher
$R^1$ und $R^2$ die oben angegebene Bedeutung haben,
mit Iso(-thio-)cyanaten der Formel III
$X=C=N\text{-}R^3$     III
in welcher
$R^3$ die oben angegebene Bedeutung hat,
umsetzt, oder
b) Verbindungen der Formel IV

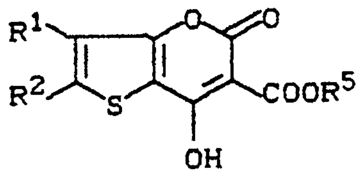

IV

in welcher
R1 und $R^2$ die oben angegebene Bedeutung haben und
$R^5$ für $C_{1-4}$-Alkyl steht,
mit Aminen der Formel V
$H\ N\ R^3\ R^4$     V
in welcher
$R^3$ und $R^4$ die oben angegebenen Bedeutungen haben,
umsetzt.

3. Verbindungen der Formel IV

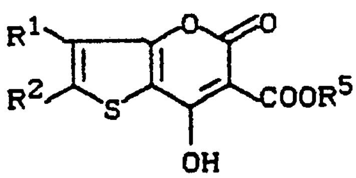

IV

in welcher
$R^1$, $R^2$, $R^5$ die in Anspruch 1 angegebenen Bedeutungen haben.

4. Verfahren zur Herstellung der Verbindungen der Formel IV gemäß Anspruch 3, dadurch gekennzeichnet, daß man Verbindungen der Formel II

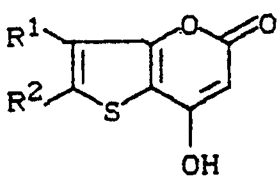

II

in welcher
$R^1$ und $R^2$ die oben angegebene Bedeutung haben,
mit Chlorameisensäureestern der Formel VI
$Cl\text{-}COOR^5$     VI
in welcher
$R^5$ für $C_{1-4}$-Alkyl steht,

umsetzt.

5. Verwendung von substituierten Thienopyranonen der Formel I gemäß Anspruch 1 zur Bekämpfung von Parasiten.

6. Endoparasitizide Mittel, gekennzeichnet durch einen Gehalt an mindestens einem substituierten Thienopyranon der Formel I gemäß Anspruch 1.

7. Verfahren zur Herstellung von endoparasitiziden Mitteln, dadurch gekennzeichnet, daß man substituierte Thienopyranone der Formel I gemäß Anspruch 1 mit Streckmitteln und/oder oberflächenaktiven Mitteln vermischt.

8. Verwendung von substituierten Thienopyranonen der Formel I gemäß Anspruch 1 zur Herstellung von parasitiziden Mitteln.